# EUROPÄISCHE PATENTANMELDUNG

(11) **EP 1 231 319 A1**
(43) Veröffentlichungstag der Anmeldung: **14.08.2002**
(21) Anmeldenummer: 02000752.2
(22) Anmeldetag: 12.01.2002
(51) Int. Cl.: D06P 5/00, D06M 23/12

(54) **Verfahren zum bereichsweisen Bedrucken eines Textilmatrials**

(30) Priorität: 09.02.2001 DE 10106596
(71) Anmelder: Deotexis Inc., New York, N.Y. 10022-4838 (US)
(72) Erfinder: Tebbe, Gerold, 98000 Monte Carlo (MC)
(74) Vertreter: Ostertag, Reinhard

(57) **Zusammenfassung**

Um ein Textilmaterial (14) bereichweise mit unterschiedlichen physikalischen und/oder chemischen Eigenschaften auszustatten, wird vorgeschlagen, diese Teilbereiche (54,56,58,60,62) im Rotationssiebdruck mit Ausrüstschichten zu versehen, die jeweils ein Bindemittel (66) und in diesem verteilte Mikrokapseln (68;74;80;82) aufweisen.

## Beschreibung

Die Erfindung betrifft ein Verfahren zum bereichsweisen Bedrucken eines Textilmateriales unter Verwendung mindestens einer Druckform gemäß dem Oberbegriff des Anspruches 1.

Derartige bekannte Verfahren dienen dazu, neutrale Textilbahnen mit einem ein- oder mehrfarbigen Farbmuster zu versehen.

Es ist ferner bekannt, Textilbahnen auszurüsten, indem man sie durch eine Lösung einer Ausrüstungsflüssigkeit hindurchbewegt.

Auch ist es bekannt, Textilmaterial mit einer Kunststofffolie zu vernähen oder zu verschweißen, die durch Sintern von Kunststoffpartikeln oder Erzeugung von Mikrorissen für Wasserdampf durchlässig, für Wasser hingegen undurchlässig ist.

Die letztgenannten beiden Verfahren erlauben es nur, dass Textilmaterial in seiner vollen Fläche mit gewünschten Eigenschaften zu versehen. Auch kann nur eine begrenzte Anzahl von Ausrüstungsmaterialien Verwendung finden.

Durch die vorliegende Erfindung soll ein Verfahren gemäß zum Oberbegriff des Anspruches 1 so weitergebildet werden, dass eine variable und flexible Bedruckung eines Textilmateriales, wie sie an sich vom Mehrfarbendruck von Textilbahnen bekannt ist, auch beim Ausrüsten eines Textilmateriales möglich ist, wobei man auch Tinten mit höherer Viskosität verarbeiten kann.

Diese Aufgabe ist erfindungsgemäß gelöst durch ein Verfahren mit dem in Anspruch 1 angegebenen Merkmalen.

Bei dem erfindungsgemäßen Verfahren macht man von der Eigenschaft des Siebdruckes Gebrauch, dass man auch verhältnismäßig viel Tinte pro Flächeneinheit auftragen kann. Man kann auch insbesondere Tinten verarbeiten, die größere Partikel enthalten. Da man Tinten mit größerer Viskosität durch eine Siebdruckform auf das Textilmaterial übertragen kann, kann man auch eine größere Anzahl von physikalischen und/oder chemischen Eigenschaften des Textilmateriales gezielt beeinflussen. In manchen Anwendungsfällen, in denen der gesteuerten physikalische und/oder chemische Effekt der Ausrüstung von der Masse des aufgetragenen Materiales abhängt, kann man mit dem erfindungsgemäßen Verfahren diesen Effekt stärker machen oder überhaupt erst in wirtschaftlich interessanter Intensität vorsehen.

Dabei läßt sich das erfindungsgemäße Verfahren mit verhältnismäßig kostengünstigen Druckmaschinen und auch in der Kleinserienfertigung (Flachsiebdruck) realisieren. Die Herstellung der Siebdruckformen ist ein dem Fachmanne bekannte Standardverfahren, welches keine Probleme bereitet.

Auch dann, wenn sich die durch das Bindemittel gebildete Ausrüstschicht beim Gebrauch des Textilmateriales verbraucht, ist es vorteilhaft, die Ausrüstschicht schon in größerer Dicke vorzusehen, wie dies mit dem erfindungsgemäßen Verfahren möglich ist.

Vorteilhafte Weiterbildungen der Erfindungen sind Gegenstand der Unteransprüche.

Mit dem Verfahren gemäß Anspruch 2 wird erreicht, dass ein Wirkstoff in die Ausrüstschicht integriert ist, der so lange inaktiv bleibt, bis er durch Zerstörung des Wandmaterials der Mikrokapseln gewollt freigesetzt wird.

Die Weiterbildung der Erfindung gemäß Anspruch 3 dient der Langzeitstabilität der Mikrokapseln.

Mit der Weiterbildung der Erfindung gemäß Anspruch 4 wird erreicht, dass sich die Mikrokapseln gut mit dem Bindemittel verbinden.

Eine besonders gute Verbindung zwischen Mikrokapselwänden und Bindemittel erhält man gemäß Anspruch 5. In diesem Falle haben auch die über die Ebene der Ausrüstschicht etwas überstehenden Abschnitte von Mikrokapseln und die von Mikrokapsel freien Abschnitte der Ausrüstschicht im wesentlichen gleiche physikalische und chemische Eigenschaften. Sind zum Beispiel Bindemittel und Wandmaterial im Hinblick darauf ausgewählt, dass sie besonders kleinen Reibungskoeffizienten zur Oberfläche der menschlichen Haut aufweisen, so hat man in allen Teilbereichen der Ausrüstschicht gleichermaßen diese Eigenschaft, die den Komfort beim Tragen des Textilmateriales auf der Haut gewährleistet.

Mit dem Verfahren nach Anspruch 6 werden Textilmaterialien hergestellt, die sich in unterschiedlichen Gebrauchseigenschaften besonders für Kleidungsstücke eignen.

Wählt man das Bindemittel der Tinte gemäß Anspruch 7, so erhält man eine besonders flexible und robuste Ausrüstschicht.

Die Weiterbildung der Erfindung gemäß Anspruch 8 stellt sicher, dass auch das Wandmaterial der Mikrokapseln gute mechanische Stabilität aufweist.

Das mit einem Verfahren gemäß Anspruch 9 hergestellte Textilmaterial zeichnet sich durch eine besonders gute Luftdurchlässigkeit aus, da der Matrixfilm porös wird, wenn das Treibmittel beim Trocknen der Tinte freigesetzt wird. Auch kann man so die Dicke der Ausrüstschicht vergrößern.

Mit einem Verfahren gemäß Anspruch 10 ist es möglich, auf einfache Weise nur Teilbereiche eines Textilmateriales unterschiedlich auszurüsten.

Als Ergebnis eines Verfahrens gemäß Anspruch 11 erhält man ein Textilmaterial, welches in seiner physikalischen und/oder chemischen Eigenschaften in unterschiedlichen Bereichen unterschiedlich gestaltet ist. Eine derartige nur bereichsweise Modifizierung physikalischer und/ oder chemischer Eigenschaften ist mit dem klassischen Ausrüstungsverfahren nicht möglich, die stets die Textilbahn als ganze betrifft.

Bei einem Verfahren gemäß Anspruch 12 kann man in einem einzigen Druckschritt mehrere unterschiedliche physikalische und/oder chemische Eigenschaften des Textilmaterials gleichzeitig vorgeben.

Das Verfahren gemäß Anspruch 13, bei welchem der Rotationssiebdruck zum Einsatz kommt, eignet sich besonders gut zur Großserienherstellung von in periodischen Strukturen ausgerüsteten Textilmaterialien.

Derartige periodische Strukturen in einer fortlaufenden Textilmaterialbahn sind insbesondere dann interessant, wenn aus dieser Textilmaterial in einem späteren Verarbeitungsschritt Zuschnitte erzeugt werden, aus denen Kleidungsstücke oder dergleichen hergestellt werden sollen. Bei dem Verfahren gemäß Anspruch 14 sind die Periode der aufgedruckten Muster sichtbarmachende Hilfsmittel, z.B. Marken, an denen die Textilmaterialbahn zerschnitten werden soll oder die Konturen eines Schnittmusters richtig auf die ausgerüsteten Bereiche der Textilmaterialbahn ausgefluchtet.

Die Weiterbildung der Erfindung gemäß Anspruch 15 gestattet es, durch die Aufrasterung die Intensität der gewünschten physikalischen und/oder chemischen Modifikation des Textilmateriales unabhängig von der Schichtdicke der Ausrüstschicht zu variieren. Dies ist insbesondere dann vorteilhaft, wenn die Ausrüstschicht eine gewisse Mindestdicke haben muss, um unter Dauereinsatzbedingungen stabil zu sein.

Die im Anspruch 16 angegebenen Rasterweiten haben sich für die Bedruckung von Textilmaterialien, die in Kleidungsstücken verwendet werden, besonders bewährt. Man hat einerseits ausreichend große Pixsel, die einen guten mechanischen Zusammenhalt von Ausrüstschicht und Textilmaterial gewährleisten. Andererseits sind diese Pixsel noch nicht so groß, dass man beim Tragen der Kleidungsstücke lokale Unterschiede in den physikalischen und/oder chemischen Eigenschaften des Textilmateriales auf der Haut realisieren würde.

Eine Rasterweite, wie s sie im Anspruch 17 angegeben ist, eignet sich insbesondere zur Herstellung von Ausrüstschichten, die für Wasserdampf durchlässig, für Wasser dagegen undurchlässig sind. Welche Rasterweite im einzelnen verwendet wird, hängt von der Art des Textilmateriales ab.

Die im Anspruch 18 angegebenen Rasterweiten eignen sich besonders für solche Ausrüstschichten, mit denen die Trageigenschaften eines Textilmateriales auf der Haut modifiziert werden sollen. Es handelt sich hierbei insbesondere um Ausrüstschichten, welche das Gleiten eines Textilmateriales auf der Haut, die Aufnahme von Feuchtigkeit, die Abgabe von Hautpflegemitteln und die Abgabe von schweißhemmenden Mitteln oder Medikamenten bewirken. Ein Textilmaterial, welches nach dem Verfahren gemäß Anspruch 18 hergestellt ist, eignet sich insbesondere auch zur Langzeitabgabe bestimmter Medikamente auf perkutanem Wege.

Erzeugt man eine Ausrüstschicht nach dem in Anspruch 19 angegebenen Verfahren, so ändert die Ausrüstschicht das optische Aussehen des Textilmaterials nur unwesentlich oder gar nicht.

Bei Anwendung eines Verfahrens gemäß Anspruch 20 kann die Ausrüstschicht zugleich eine optische Sperrschicht bilden. Dies kann insbesondere für dünne Stoffe wie Blustoffe von Interesse sein.

Die nach dem Verfahren gemäß Anspruch 21 erzeugte Ausrüstschicht ist lichtundurchlässig und weiß, während eine nach dem Verfahren gemäß Anspruch 22 erzeugte Ausrüstschicht bewußt farbig gestaltet werden kann, damit sie entweder in der Farbe genau zur Farbe des Textilmateriales passt oder zu dieser Farbe einen gewünschten farblichen Kontrast sicherstellt.

Nachstehend wird die Erfingung anhand eines Ausführungsbeispieles unter Bezugnahme auf die Zeichnung näher erläutert. In dieser zeigen:
- Figur 1:: eine schematische Darstellung einer Mehrstationen-Siebdruck-Rotationsmaschine, die zum Bedrucken einer Textilmaterialbahn dient; und
- Figur 2:: einen Ausschnitt aus der bedruckten Textilmaterial bahn.

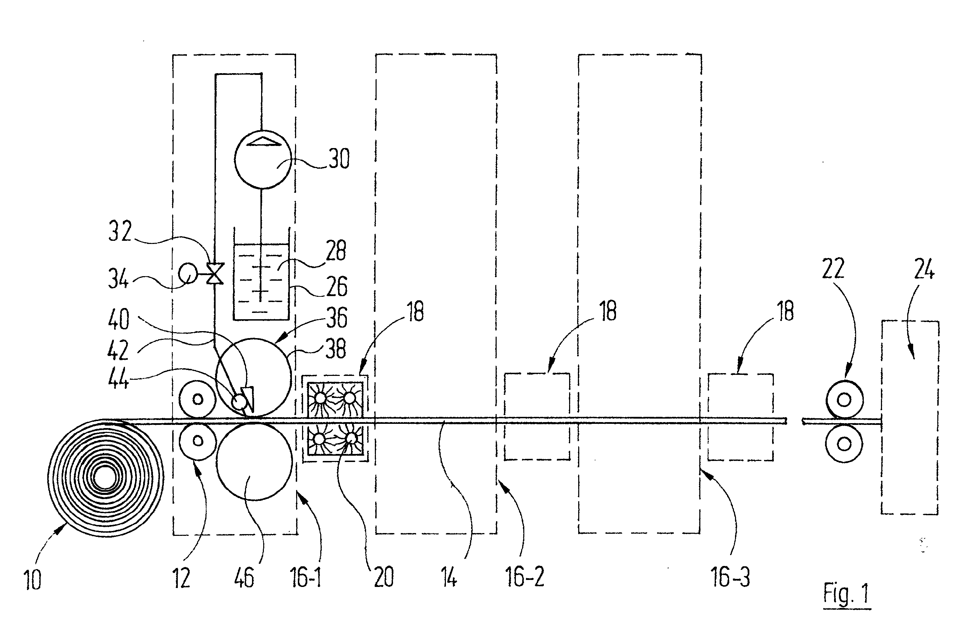

In Figur 1 ist mit 10 eine Vorratsrolle bezeichnet, von welcher durch ein Transportwalzenpaar 12 eine Textilmaterialbahn 14 abgezogen wird. Bei der Textilmaterialbahn 14 handelt es sich in der Regel um ein Gewebe, es können jedoch auch andere Textilmaterialbahnen verwendet werden, z.B. Gewirke und Fliese. Diese verschiedenen Textilmaterialbahnarten können aus natürlichen Fasern, Kunstfasern oder Mischungen dieser beiden Faserarten bestehen. Für die Zwecke der vorliegenden Beschreibung sollen auch Kunststofffolien und Papierarten, die für die Herstellung von Kleidungsstücken in Betracht kommen, mit unter dem Begriff Textilmaterialbahn verstanden werden.

Die Textilmaterialbahn 14 wird durch verschiedene in Förderrichtung aufeinanderfolgende Druckstationen 16-1, 16-2,16-3 oder allgemein 16-i hindurchbewegt, die nachstehend genau beschrieben werden. Zwischen den einzelnen Druckstationen befindet sich jeweils eine Trockenstation 18, die beim hier betrachteten Ausführungsbeispiel Strahlerstäbe 20 aufweisen. Je nach verwendeter Tinte kann es sich um IR-Strahlerstäbe, UA-Strahlerstäbe oder Kombinationen dieser Strahlerstäbe handeln. Auch eine Heißlufttrocknung kommt in den Trockenstationen 18 in Betracht.

Hinter der letzten Trockenstation wird die Textilmaterialbahn 14 von einem weiteren Transportwalzenpaar 22 übernommen, welche die Textilmaterialbahn zu einer schematisch angedeuteten Schneidstation 24 weiterfördert. In dieser wird die bedruckte Textilmaterialbahn 14 in einzelne Abschnitte zerlegt, wie dies der Periodizität des aufgebrachten Druckmusters entspricht. Zusätzlich können dort die Bahnabschnitte einzeln oder nach Sammeln in einem Stapel zu Zuschnitten geformt werden, die Teilen eines Kleidungsstückes entsprechen, z.B. Teile einer Hose, Teile eines Hemdes, Teile einer Jacke usw.

In den verschiedenen Druckstationen 16 wird auf die Textilmaterialbahn 14 jeweils eine Tinte aufgetragen. Hierzu hat jede Druckstation einen Vorratsbehälter 26, in welchem sich eine Tinte 28 befindet. Eine Förderpumpe 30 saugt die Tinte aus dem Vorratsbehälter 26 an und gibt sie über ein Dossierventil 32, welches durch einen Servomotor 34 verstellt wird, ins Innere einer Siebdrucktrommel 36. Diese hat eine als Siebdruckschablone ausgebildete Umfangswand 38, auf deren Innenseite ein Rakel 40 läuft, wie dargestellt. Eine Speiseleitung 42, die mit dem Ausgang des Dossierventiles 32 verbunden ist, ist mit einem Verteilohr 44 verbunden, welches in Drehrichtung der Siebdrucktrommel 38 gesehen stromauf des Rakels 40 liegt und dort über die axiale Länge der Siebdrucktrommel gleiche Mengen an Tinte abgibt.

Unter der Siebdrucktrommel 36 ist eine Gegentrommel 46 angeordnet, die eine nachgiebige weiche Umfangsfläche hat und synchron zur Siebdrucktrommel 36 angetrieben wird, wie dem Fachmanne bekannt.

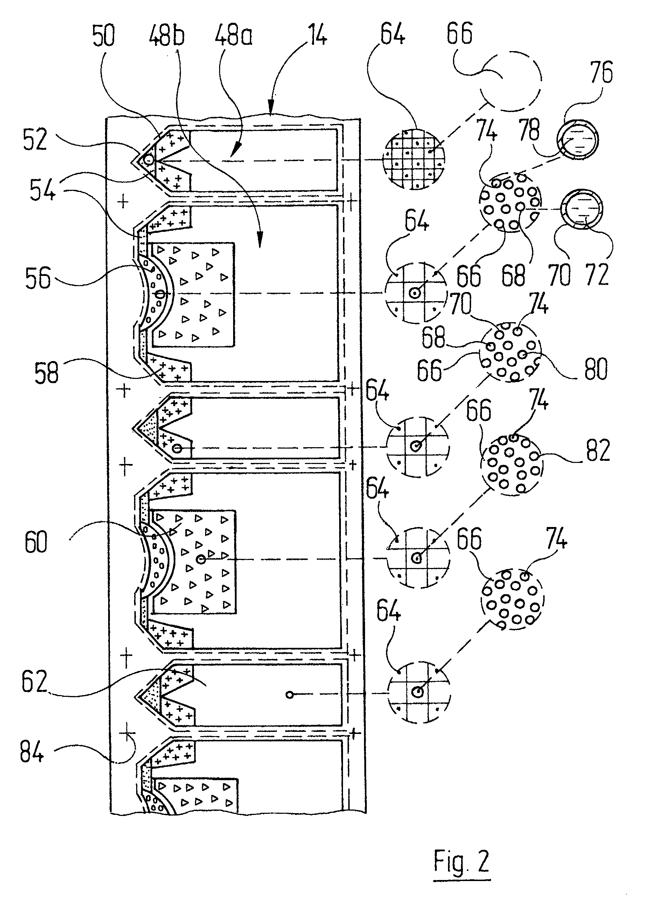

Figur 2 zeigt einen Ausschnitt aus der bedruckten Textilmaterialbahn, wobei zu Erläuterungszwecken angenommen ist, dass aus der Textilmaterialbahn 14 Zuschnitte zur Herstellung von T-Shirts hergestellt werden sollen.

Die insgesamt mit 48a und 48b bezeichneten Zuschnitte für Ärmel bzw. Shirt-Hauptteile, von denen der Einfachheit halber angenommen werden soll, dass gleiche Zuschnitte für Vorderseite und Hinterseite des T-Shirts verwendet werden sollen, haben eine Randkontur 50, welche durch Schneiden der Textilmaterialbahn in der Schneidstation 22 sichtbar wird, auf der bedruckten Materialbahn aber nicht augenfällig in Erscheinung tritt. Falls die Schnittlinie visualisiert werden soll, kann man in einer letzten Druckstation 16 eine der Randkontur 50 und in kleinem Abstand folgende Schnittlinie 52 mit farbiger Tinte aufdrucken.

Im Bereich der Zuschnitte 48 kann man verschiedene Ausrüstbereiche unterscheiden, welche jeweils aus der Zeichnung ersichtliche Randkontur haben und durch kleine Markierungsmuster unterschieden sind: Einen ersten Ausrüstbereich 54 im späteren Schulterbereich, der eine Ausrüstschicht aufweist, die wasserundurchlässig ist; einen zweiten, dem Ausschnitt benachbarten Ausrüstbereich 56, der mit einer duftstoffabgebenden Ausrüstschicht versehen ist; einen dritten Ausrüstbereich 58, der den Achselabschnitten des fertigen T-Shirts zugeordnet ist und Wirkstoffe enthält, welche die Schweißbildung und/oder Schweißzersetzung hemmen (Zusätzlich kann dieser Ausrüstbereich mit einem Deodorant ausgestattet sein.); einen weiteren Ausrüstbereich 60, der im fertigen T-Shirt den Rücken bzw. dem Brustkorb zugeordnet ist und mit einer Ausrüstschicht versehen ist, welche sowohl feuchtigabsorbierende Mittel als auch hautpflegende Mittel aufweist und zudem so ausgestattet ist, dass sie leicht auf der Hautoberfläche gleitet; und einen den Rest des Zuschnittes im wesentlichen überdeckenden weiteren Ausrüstbereich 62 mit einer Ausrüstschicht, welche hautpflegende Wirkstoffe enthält und auf der Hautoberfläche gut gleitet.

Wie für die Ausrüstbereiche 54, 56, 58, 60, 62 dargestellt, sind die verschiedenen Tinten, welche die Ausrüstschichten bilden, im Rasterdruck aufgebracht worden. Mit Tinte bedruckte Rasterelemente sind in der Zeichnung durch einen Punkt markiert. Die einzelnen Rasterelemente 64 haben eine Kantenlänge, die in der Praxis je nach Art des Textilmateriales zwischen etwa 0,1 und etwa 10 mm beträgt.

Beim Ausrüstbereich 54, bei welchem auf eine wassersperrende Wirkung abgehoben wird, liegt die Kantenlänge des Rasterelementes im Bereich zwischen 0,1 und 0,5 mm. Für feine Gewebe wie Hemdenstoffe wird eine Kantenlänge im Bereich von 0,1 bis 0,3 mm gewählt. Für gröbere Textilmaterialien, z.B. ein dünnes Gestrick wir es für T-Shirts verwendet wird, kann die Kantenlänge des Rasterelementes eher im oberen Bereich von etwa 0,3 bis 0,5 mm gewählt werden.

Die Tinte, die zum Drucken des Ausrüstbereiches 54 verwendet wird, kann eine reine, von Fremdkörpern freie Silicon-Tinte (reine Matrixschicht 66) sein wie aus der weiteren Ausschnittvergrößerung ersichtlich. Die aus dieser Tinte erhaltene Ausrüstschicht ist gut flexibel und sperrt Wasser.

Im Ausrüstbereich 56 hat man wieder ein Druckmuster mit Rasterelementen 64, wobei nun aber die Kantenlänge der Rasterelemente etwas größer gewählt ist, z.B. im Bereich zwischen 1 und 3 mm. Man hat so etwas größere zwischen den Rasterelementen liegende Materialbereiche, die vollständig tintenfrei ist. Dies ist im Hinblick auf eine gute Luftdurchlässigkeit des fertigen Produktes wünschenswert, wobei die Ausrüstschicht aber vom Benutzer nach wie vor als homogen empfunden wird.

Wie aus der zweiten Ausschnittvergrößerung ersichtlich, haben die Rasterelemente 64 der Ausrüstschicht 56 jeweils eine Matrix 66, in welche Mikrokapseln 68 eingebettet sind. Die Matrix besteht aus einem Silicon-Mittel, die Mikrokapseln 68 haben jeweils eine Wand 70, die ebenfalls aus einem Silicon-Material besteht, und einen Wirkstoff 72, in welchem ein Duftstoff eingeschlossen ist. Das Material der Wand 70 ist so gewählt, dass bei Temperaturerhöhung kleine Mengen des Wirkstoffes 72 (Duftstoff) durch die Wand hindurchtreten kann.

Die Rasterelemente 64 enthalten ferner weitere Mikrokapseln 74, die eine Wand 76 haben, die ein Hautpflegeöl 78 umgibt. Wird die Wand 76 beim Tragen des T-Shirts aufgerieben, so wird das Hautpflegeöl 78 sukzessive abgegeben.

Im Ausrüstbereich 58 hat man wiederum Rasterelemente 64, deren Größe ähnlich gewählt sein kann wie im Ausrüstbereich 56. Im Bindemittel 66 sind nun aber neben den Mikrokapseln 68 und 74 noch weitere Mikrokapseln 80 enthalten, die die Schweißbildung und/oder die Schweißzersetzung hemmende Wirkstoffe enthalten.

Im ähnlich aufgebauten Ausrüstbereich 60 hat man im Bindemittel 70 Mikrokapseln 74, die ein Hautpflegeöl enthalten, so wie weitere Mikrokapseln 82, die ein Feuchtigkeit absorbierendes Material enthalten.

Im restlichen Ausrüstbereich 62 enthalten die Rasterelemente 64 im Bindemittel 66 nur die Hautpflegeöl enthaltenden Mikrokapseln 74.

Auf diese Weise sind die unterschiedlichen Teilbereiche der Zuschnitte 48 und damit auch des hieraus hergestellten T-Shirts unterschiedlich ausgerüstet, wie dies im Hinblick auf die mit ihnen in Kontakt kommenden Bereiche der Hautoberfläche wünschenswert ist.

Die mit den entsprechenden Tinten gedruckten Bereich der Zuschnitte 48 unterscheiden sich optisch nur wenig von der Textilmaterialbahn 14. Um die verschiedenen Abschnitte 26 der Textilmaterialbahn zum Zuschneiden registergerecht übereinanderlegen zu können, werden in einer letzten Druckstation 16, die mit einer pigmentierten Druckfarbe arbeitet, Marken 84 auf die Textilmaterialbahn 14 gedruckt, die das ausgefluchtete übereinander liegende Bahnabschnitte ermöglichen und beim Zuschneiden wegfallen.

Für andere Einsatzzwecke können abgewandelte Tinten verwendet werden, die andere Mikrokapseln enthalten, die einen oder mehrere der nachstehenden Stoffe umschließen: Medikamente, Nahrungsergänzungsmittel, insbesondere Vitamine, und die Temperatur stabilisierende Materalien.

Beim oben beschriebenen Ausführungsbeispiel wurde davon ausgegangen, dass für alle Ausrüstbereiche dasselbe Bindemittel verwendet wird. Dies ist im Hinblick auf überall gleiche Trageingenschaften wünschenswert. Insbesondere werden Silicon-Bindemittel bevorzugt, da diese leicht auf der Haut gleiten.

In Abwandlung des obigen Ausführungsbeipieles kann man auch eine Tinte verwenden, bei der im Bindemittel Mikrokapseln enthalten sind, die ein Treibmittel enthalten und deren Wandmaterial bei Wäremeinwirkung aufgeht. Beim Trocknen der Tinte wird dann das Treibmittel freigesetzt. Dies kann, je nach Art des Bindemittels, dazu führen, daß das Bindemittel eine verglichen mit der Tintenschicht dicke Schaumschicht bildet, oder dazu, daß die getrocknete Tintenschicht kleine Löcher aufweist.

Wo aber in einzelnen Ausrüstbereichen besondere Eigenschaften gefordert werden, die Vorrang haben, kann in kleinen Bereichen wie z.B. im Ausrüstbereich 54 auch ein unterschiedliches Bindemittel verwendet werden, um dort spezielle Eigenschaften zu gewährleisten. Auf die Komplexität und die Kosten des Herstellungsverfahrens hat dies keinen Einfluß.

## Patentansprüche

1. Verfahren zum bereichsweisen Bedrucken eines Textilmaterials (14) unter Verwendung mindestens einer Druckform (36), bei welchem die Druckform (36) mit einer Tinte versorgt wird und die mit Tinte versorgte Druckform in Kontakt zum Textilmaterial (14) gebracht wird, **dadurch gekennzeichnet, daß** die Druckform (36) eine Siebdruckform ist und daß mindestens eine der Tinten ein Bindemittel (66) und einen von diesem getragenen Wirkstoff (68; 74; 80; 82; 84) aufweist.

2. Verfahren nach Anspruch 1, **dadurch gekennzeichnet,**
**daß** mindestens einer der Wirkstoffe (68; 74; 80; 82) mikroverkapselt verwendet wird.

3. Verfahren nach Anspruch 2, **dadurch gekennzeichnet,**
**daß** ein Wandmaterial der Mikrokapseln (68; 74; 80; 82; 84) gegen das Bindemittel (66) stabil ist.

4. Verfahren nach Anspruch 3, **dadurch gekennzeichnet,**
**daß** das Bindemittel (66) und das Wandmaterial (70; 76) der Mikrokapseln (68; 74; 80; 82) chemisch verwandt sind.

5. Verfahren nach Anspruch 4, **dadurch gekennzeichnet,**
**daß** das Bindemittel (66) und das Wandmaterial (70; 74) im wesentlichen durch das gleiche Kunststoffmaterial gebildet sind.

6. Verfahren nach einem der Ansprüche 2 bis 5, **dadurch**
**gekennzeichnet, daß** die Mikrokapseln (68; 74; 80; 82) einen oder mehrere der nachstehenden Stoffe umschließen: Feuchtigkeitsabsorptionsmittel, Hautpflegemittel, Medikamente,, Nahrungsergänzungsmittel, insbesondere Vitamine, die Schweißbildung hemmende oder schweißzersetzende Wirkstoffe, die Temperatur stabilisierende Materalien, Duftstoffe.

7. Verfahren nach einem der Ansprüche 1 bis 6, **dadurch**
**gekennzeichnet, daß** das Bindemittel (66) ein Silikonmaterial ist.

8. Verfahren nach einem der Ansprüche 2 bis 7, **dadurch**
**gekennzeichnet, daß** das Wandmaterial (70; 76) der Mikrokapseln (68; 74; 80; 82) ein Silikonmaterial ist.

9. Verfahren nach einem der Ansprüche 1 bis 6, **dadurch**
**gekennzeichnet, daß** die Tinte ein vorzugsweise mikroverkapseltes Treibmittel enthält, wobei das Wandmaterial der Mikrokapseln durch Wärmeeinwirkung beim Trocknen zerstörbar ist.

10. Verfahren nach einem der Ansprüche 1 bis 9, **dadurch**
**gekennzeichnet, daß** eine Mehrzahl aufeinanderfolgender Druckschritte in unterschiedlichen Teilbereichen (54-62) der Textilmaterialbahn (14) erfolgt.

11. Verfahren nach Anspruch 10, **dadurch gekennzeichnet,**
**daß** sich die einzelnen Teilbereiche (54-62) nicht überlappen.

12. Verfahren nach einem der Ansprüche 1 bis 11, **dadurch**
**gekennzeichnet, daß** die Tinte eine Mischung unterschiedlicher Wirkstoffe umfaßt.

13. Verfahren nach einem der Ansprüche 1 bis 12, **dadurch**
**gekennzeichnet, daß** eine zylindrische umlaufende Siebdruckform (36) verwendet wird.

14. Verfahren nach einem der Ansprüche 1 bis 13, **dadurch**
**gekennzeichnet, daß** auf die Textilmaterialbahn (14) die Randkontur (52) eines Zuschnitts aufgedruckt wird.

15. Verfahren nach einem der Ansprüche 1 bis 14, **dadurch**
**gekennzeichnet, daß** die Tinte im Raster-Siebdruck aufgedruckt wird.

16. Verfahren nach Anspruch 15, **dadurch gekennzeichnet,**
**daß** Rasterelemente (66) der Siebdruckform (36) eine Abmessung von etwa 0,1 bis etwa 10 mm aufweisen.

17. Verfahren nach Anspruch 16, **dadurch gekennzeichnet,**
**daß** die Rasterelemente (66) der Siebdruckform (36) eine Abmessung zwischen etwa 0,1 und etwa 1 mm, vorzugsweise zwischen etwa 0,1 und etwa 0,5 mm aufweisen.

18. Verfahren nach Anspruch 16, **dadurch gekennzeichnet,**
**daß** die Rasterelemente (66) der Siebdruckform (36) eine Abmessung zwischen etwa 0,3 mm und etwa 6 mm, vorzugsweise zwischen etwa 1 mm und etwa 3 mm aufweisen.

19. Verfahren nach einem der Ansprüche 1 bis 18, **dadurch**
**gekennzeichnet, daß** das Bindemittel (66) durchsichtig oder durchscheinend ist.

20. Verfahren nach einem der Ansprüche 1 bis 18, **dadurch**
**gekennzeichnet, daß** das Bindemittel (66) pigmentiert ist.

21. Verfahren nach Anspruch 20, **dadurch gekennzeichnet,**
**daß** die Pigmente weiß sind.

22. Verfahren nach Anspruch 20, **dadurch gekennzeichnet,**
**daß** die Pigmente farbig sind.
